# EUROPEAN PATENT APPLICATION

(11) **EP 1 147 767 A1**
(43) Date of publication of application: **24.10.2001**
(21) Application number: 00108740.2
(22) Date of filing: 22.04.2000
(51) Int. Cl.: A61K 9/20, A61K 31/63, A61P 29/00

(54) **Controlled release formulations of nimesulide and a process for the manufacture thereof**

(71) Applicant: J.B. Chemicals & Pharmaceuticals Ltd., Mumbai 400 025, Maharashtra (IN)
(72) Inventor: Doshi, Madhukant Mansukhlal, Dr., Mumbai 400 077, Maharashtra (IN); Joshi, Milind Dattatraya, Dr., Thane 400 604, Maharashtra (IN); Mody, Shirish Bhagwanlal, Mumbai 400 006, Maharashtra (IN)
(74) Representative: Tergau & Pohl Patentanwälte

(57) **Abstract**

A controlled release formulation in the form of oral dosage for treating inflamatory and pain disorders by administering Nimesulide in combination with hyprohilic matrix forming polymer to produce granules together with pharmaceutically acceptable excipients including binders and lubricants, tableted as per the commonly used pharmaceutical technology, said oral formulation provides a drug release of nimesulide for extended period of 24 hours.

## Description

### FIELD OF THE INVENTION

The present invention relates to the preperation of controlled release formulations of Nimesulide which provides the control release of active drug over a desired period in the treatment of various type of inflammatoion and pain disorders and a process for the preperation thereof.

### BACKGROUND OF THE PRIOR ART

Nimesulide is NSAID (Non-steroidal anti-inflammatory drug) of the sulphonanilide class. Chemically it is 4'-Nitro-2'-phenoxymethanesulphonanilide. Nimesulide is reported to be a selective inhibitor of cyclo-oxygenase-2.

Nimesulide, unlike other NSAIDs does not inhibit both COX-1 and COX-2 enzymes. It is selective inhibitor of COX-2 enzyme. It is a known fact that COX-1 enzyme helps to maintain the stomach lining and COX-2 enzyme is involved in the pain and inflammation but the durgs which inhibits both the enzymes may damage the stomach lining and thus it may lead to ulcers. Whereas, Nimesulide reduceses the gastrointestinal side effects including dyspepsis, diarrhea and abdominal pain associated with other NSAIDs as it inhibits only COX-2.

Compared to other NSAID's Nimuesulide has a favorable therapeutic index with minimal gastrointestinal toxicity. Nimesulide has antipyretic and analgesic properties. It seems to have a lower propensity than other NSAIDs for inducing allergic reactions and obstructive pulmonary reactions in patients with asthama and in aspirin and/or NSAID intolerent patients.

Nimesulide has exhibited potency similar to or greater than that of indomethacin, diclofenac, piroxicam and ibuprofen in standard animal model of inflammation. Moreover it has shown superior antipyretic potency to indomethacin, ibuprofen, aspirin and paracetamol in rats. Animal model suggests that nimesulide is less ulcerogenic than aspirin, indomethacin, naproxen, piroxicam and ibuprofen.

Nimesulide is very hydrophobic drug and practically insoluble in water. Its aqueous solubility is about 0.01 mg/ml. Poor aqueous solubility presents the problem for the preperation of pharmaceutical formulation with good release and bioavailability. Efforts have be made to enhance the solubility by producing solid dispersion in hydropillic carriers (US Appl. 5776495 ) and by forming inclusion complex with betacyclodextrine (US Appl. 5744165).

Nimesulide is a weak acid type compound, therefore its aqueous solubility in acidic medium (gastric juice) is poor. Nimesulide is a selective inhibitor of prostaglandin synthesis and appears to exert its effects through a variety of mechanisms including free radical scavenging, on histamine release, the neutrophil myeloperoxideas pathway, bradykinin activity, tumor necrosis factor-alpha release, phosphodiesterase type VI inhibition, inhibition of platelet aggregation and synthesis of platelet activating factor.

Nimesulide showed efficacy in the treatment of pain and inflammation associated with rheumatoid arthritis and osteoarthritis. Nimesulide 200 mg/day was generally more effective than the usual therapeutic doses of naproxen, ibuprofen, ketoprofen mefenamic acid and suprofen in reducing clinical sysmtoms associated with variety of conditions including respiratory tract infections, ear, nose and throat disorders, dysmenorrhoea, dental surgery, urogentital tract disorders and musculoskeletal injuries.

Nimesulide is conventionally administered in tablet form of 100 mg/day to 400 (mostly 200 mg/day) for 5 to 21 (mean 12 days). Conventional Nimesulide therapy starts with 100 mg adminstrered twice daily. The dosage is gradually increased to 100 mg administratered four times a day until optimum response is obtained.

The oral and rectal dosages of nimesulide in adults are 100 and 200 mg twice daily respectively. In children, nimesulide dosage is commonly 5 mg/kg/day divided in 2 or 3 daily doses administered in the form of suspension, granules or suppositories.

As stated hereinabove, conventional Nimesulide tablets are administered two or four times daily. Such a frequent drug adminstration may reduce patient compliance, there is possiblity of exhibiting toxic effects accompained by side effect.

However, Nimesulide is also required to be given in higher doses of 400 mg and 600 mg / day in post operative pain providing moderate relief to the pain. Nimesulide also is topically applied at the affected area for releving post operative pain (US Appl. 5998480). Moreover, transdermal drug delivery system (US Appl. 6024,976), formulations in form of thixotropic gel (US Appl. 5858330) and composition containing nimesulide and piperine (US Appl. 6017932) to enhance therapeutic efficacy have been disclosed in the references stated hereinabove.

Earlier studies reported that in a double-blind study in the patient for saphenectomy or inguinal hernioplasty were randomly assigned to the treatment with nimesulide (200mg 3 times daily) administered rectally. This therapy resulted in significantly less pain, oedema and hyperaemia and resolution of mild fever and no adverse reaction was attributated by Ramella, G. et. al. (Drugs 46 (Suppl. 1), 159-161, 1993).

In Coscarelli, S. et.al. (Drugs 46, (suppl. 1) 174-176, 1993), it is disclosed that Nimesulide (200 mg twice daily) when admistered rectally gave relief in the pain associated with postoperative inflammatory complications of ear, nose and throat surgery. Moreover Pierleoni, P. et. al. (Drugs 46, (suppl. 1) 168-170, 1993) also disclosed that nimesulide gave relief when adminstered at the same doses of 200 mg twice daily in dental surgery. Toscani, F. et. al. (Drugs 46 (Suppl. 1) 156-158, 1993) reported analgesic efficacy of nimesulide with advanced cancer who needed to be treated with NSAID drug according to the first step of the pharmacological analgesic scale of the WHO. Patient receiving 200 mg twice dalily administered as granular formulation. This therapy resulted in similarly reduced pain with naproxen 500 mg twice daily.

As disclosed earlier, Nimesulide is frequently adminstered, such frequent drug administration may reduce patient compliance resulting thereby every possiblity of exhibiting toxic effect accompanied by side effect. To attain and maintain a blood level which exceeds the minimum effective level required for response, but does not exceed the minimum toxic level, the dose of 200 mg in controlled release formulation is convenient. In the cases where special indications need i.e in saphenectomy or inguinal hernioplasty the 400 mg tablet with controled release profile can be convenient for once a day administration.

It is frequently desirable to delay the release of an active substance from a pharmaceutical formulation in vivo. It may be desirable to delay release of active substance within the body so that the active substance is released at a particular target site. Various controlled absorption pharmaceutical formulations are available which have a particular dissolution pattern, resulting in a controlled absorption of the active substance, and therefore more medication.

Knowing the state of the art and knowing the need which has existed for years to find a formulation which has rapid onset of action and a long duration of action of this active compound nimesulide has been developed according to the invention which so far as known, no one has heretofore formulated.

The inventors of the present invention have come out with simple and inexpensibe way of producing once-a-day extended release Nimesulide tablet. It leads to good therapeutical effectiveness in patients with severe chronic pain as its action is of long duration. The present invention not only provides an effective relatively low dose treatment of various types of inflamation and pain disorders but also helps to maintain patent complaiance.

### OBJECTS OF THE INVENTION

1. It is the obect of the invention to develop once a day extended release Nimesulide tablet which exhibits controlled release characteristics.
2. It is further object of the invention to provide controlled absorption of Nimesulide formulation suitable for once-a-day administration which is particularly effective for 12 hrs to 24hrs.
3. It is still a further object of the invention to develop a NSAID formulation maintaining a high anti-inflammatory activity with less or negligible side effects.

### BRIEF DESCRIPTION OF THE INVENTION

The present invention provides a controlled release Nimesulide formulation in the form of oral dosage for treating various type of inflammation and pain disorders and the process for the preperation thereof.

Presently Nimesulide is administered frequently in the dosage form of 100, 200 mg twice a day for 12 days (mean). If the patient complaince is compormised and if the patient fails to take the second or third dose as prescribed the efficacy of the treatment may greatly impaired.

A drug release study was also performed to confirm in-vito dissolution. Samples were drawn at different time intervals and results obtained were tabulated below . An in vitro trials and clinical trials have shown that the formulation prepared by this method shown controlled effect and constant rate of absorption for desired period of time, ensures maintaing the degree of bioavailability.

The Nimesulide controlled release tablets 200 mg as prepared by example 1, 2, 3 and 4 and tablets of 400mg as prepared by example 5,6,7 and 8 of the present invention was subjected to *in vitro* drug release study. Dissolution studies were carried out as per USP type II apparatus. Dissolution medium used were alkaline borate buffer (pH 8.4) and assyed for nimesulide by measuring absorbance at 230 nm.

The results of 200 mg tablets are given below :

| **Time (Hrs)** | **% DRUG RELEASE** | | | |
|---|---|---|---|---|
| | **Example 1** | **Example 2** | **Example 3** | **Example 4** |
| **1** | 6.53 | 7.26 | 6.85 | 6.94 |
| **4** | 26.18 | 29.31 | 23.99 | 24.76 |
| **12** | 69.28 | 62.89 | 68.91 | 60.54 |
| **24** | 97.24 | 97.35 | 96.86 | 93.92 |

The results of 400 mg tablets are given below :

| **Time (Hrs)** | **% DRUG RELEASE** | | | |
|---|---|---|---|---|
| | **Example 5** | **Example 6** | **Example 7** | **Example 8** |
| **1** | 5.94 | 6.76 | 5.87 | 5.99 |
| **4** | 31.26 | 33.13 | 30.65 | 29.49 |
| **12** | 66.54 | 64.73 | 70.12 | 68.12 |
| **24** | 96.25 | 94.73 | 89.21 | 90.17 |

The results of the above studies indicates that all the Examples product clearly showed the control release profile. The tablets of 200 mg prepared as per Example 2,3 & 4 exhibited similar in-vitro dissolution rate as that of example 1. Tablet of 400 mg also exhibited controlled release profile.

The controlled release nimesulide formulation of the invention may be prepared by the following process-To a weighed quantity (taken proportionately relative to the dose of the active drug in the final formulation) of Nimesulide are added required quantity of the hydrophilic matrix forming polymer and the other necessary excipients, and the mass is thoroughly mixed and blended to make uniform paste, which is thereafter dried granulated and compressed in a conventional manner.

Hydrophillic matrix forming polymer used in this specification refers to any one or more of the pharmaceutically accepted inert polymer or mixture of such polymers normally employed and described in literature for coating of drugs for controlled release, out of which, the polymers alkylcelluloses such as methylcellulose and ethylcellulose, polyvinylpyrrolidone, hydroxymethylcellulose, hydroxyehtylcellulose, hydroxypropylcellulose, hydroxypropylmethyl cellulose, (available Hydroxypropylmethylcellulose under commercial names Methocel can be used of various grades ranging from 4000 cps to 100,000 cps like K15M, K100M, K4M alone and in combination),and sodium carboxymethylcellulose. The other polymers which may also be used for the process are polyethylene glycol, modified polymethacrylate copolymers etc.

The best results are obtained by using the preferred polymers mentioned above with a more preferred group of polymers being the cellulose polymers, like hydroxymethylcellulose, hydroxypropylmethyl cellulose, hydroxypropyl cellulose, and sodium carboxymethylcellulose, with hydroxypropylmethyl cellulose being particularly preferred. Ratio of nimesulide and hydroxypropylmetnylcellulose ranges between 1:0.1 to 1:0.3.

Moreover, hydroxypropylmehtylcellulose can be replaced by other polymers or a mixture of polymers such as mehtylcellulose, hydroxyethylcellulose and the like.

Inert excipients like lactose and microcrystalline· cellulose can also be replaced with glucose, sucrose, mannitol, galctose, sorbitol or dextrose. Ratio between nimesulide and inert excipient varies between 1:0.2 to 1:0.5

The excipients required to be used at various stages of the process are selected from pharmaceutically acceptable excipients normally employed in pharmceutical formulation. Those skilled in the art can decide as to the particular excipients and quantites thereof to be employed in the process, having regard for the quantity of the active drug to be taken and rates and periods of release of the active drug desired in the final formulation.

It will be appreciated from whatever stated above, clearly shows that Nimesulide can be administered in the oral dosage forms, topical routes in the form of gel and transdermal delivery. The formulation of the invention can also be in any form commonly employed for adminstration i.e. suspension, caplets, powders lozenges, solutions, granules, capsule, waffers, and chewables. Such formulation can be administered orally or by intramascular route. They can also be administered in the form of modified release, sustained release, controlled release, timed release formulation. They can also be adminstered by ocular, intranasal, buccal, sublingual, rectal, vaginal and other related administration routes.

The formulation obtained by the present invention will be more fully understood from the following examples. These examples are to be construed as illustrative of the invention and not limitative thereof:

### Example 1:

Nimesulide (100 Kg), lactose (39.25 Kg), Hydroxpropylmethylcellulose K15M (15 Kg) and microcrystalline cellulose (39.75 Kg) was sifted and mixed thereafter granulation was carried out by the addition of starch paste (9 Kg). Wet granules were passed through sieve, dried, and passed again through sieve. The granules thus obtained were blended with Magnesium stearate (2.5 Kg), collidal silicone dioxide (0.375 Kg) and talc (4.125 Kg) and compressed to obtain tablets containing 200 mg of Nimesulide. Tablets were then film coated using hydroxypropylmethylcellulose as a coating polymer with characteristic cosmetic colour and polished with usual coating technology.

### Example 2 :

The procedure employed was similar to that in Example 1, except that the Hydroxypropylmethylcellulose K15M (15 Kg) was replaced with Hydroxypropylmethylcellulose of K100M (10Kg) and quantity of microcrystalline cellulose was changed to 44.75 Kg.

### Example 3 :

The procedure employed was similar to that in Example 1, except that the Lactose was replaced with Mannitol.

### Example 4 :

The procedure employed was similar to that in Example 1, except that the Hydroxypropylmethylcellulose K15M (15 Kg) was replaced with Sodium carboxymethylcellulose (15 Kg).

### Example 5 :

The procedure employed was similar to that in Example 1, except that the tablet of 400 mg was produced during compression. Here, ratio of the active drug and inert exipient was maintained as per the Example 1.

### Example 6 :

The procedure employed was similar to that in Example 1, except that the Hydroxypropylmethylcellulose K15M (15 Kg) was replaced with Hydroxypropylmethylcellulose of K100M (10Kg) and quantity of microcrystalline cellulose was changed to 44.75 Kg and compressed to obtain tablets containing 400 mg of Nimesulide.

### Example 7 :

The procedure employed was similar to that in Example 1, except that the Lactose was replaced with Mannitol and compressed to obtain tablets containing 400 mg of Nimesulide.

### Example 8 :

The procedure employed was similar to that in Example 1, except that the Hydroxypropylmethylcellulose K15M (15 Kg) was replaced with Sodium carboxymethylcellulose (15 Kg) and compressed to obtain tablets containing 400 mg of Nimesulide.

### Clinical Trial:

To investigate the effectiveness of the method of this invention for the treatment of inflamatory and pain disorders, the following clinical trial were conducted.

50 patients suffering from Non articular soft tissue rheumatism between the age group of 18 to 60 were included in the study. The average age was 36.72 years, mazimum age was 52 years and minimum was 18 years. 22 patients were suffering from low back pain, 18 had posttraumatic myalgia and 10 patients had tendinitis. All the patients were evaluated for pain, tenderness, erythema, swellind, restriction of movement on day 0, day 5, day 10, and day 15. All the patients were given one tablet of 200mg this invention daily. In the present study, one tablet of present invention daily effectively reduced pain, tenderness, erythema, swelling and restriction of movement effectively as well as significantly (p < 0.01) when compaired between day 0 and day 15. The tolerability in present trial was good. No patient discontinued the medication due to side effect. Tablets of this inventin is thus an effective and well tolerated choice for the treatment of soft tissue rheumatism.

It is to be understood that the examples and embodiments described hereinabove are for the purposes of providing a description of the present invention by way of examples and are not to be viewed as limiting the present invention in any way. Modification that may be made to that described in above examples by those of ordinary skill in the art are also contemplated by the present invention and are to be included within the spirit and purview of this application and the appended claims.

## Claims

1. A controlled release formulation in the form of oral dosage form for treating inflamatory and pain disorders by administering Nimesulide in combination with hydrohilic matrix forming polymer to produce granules together with pharmaceutically acceptable excipients including binders and lubricant tableted as per the commonly used pharmaceutical technology, said oral formulation provides a drug release of nimesulide for extended period of 24 hours.

2. The formulation according to claim 1 wherein said hydrophillic matrix forming polymer is selected from alkyl-cellulose ; hydroxymethylcellulose, hydroxyethylcellulose, hydroxypropylcellulose, hydroxypropylmethylcellulose and sodiumcarboxymethyl cellulose, polyvinylpyrrolidone and mixture thereof.

3. A formulation according to claim 1, wherein said hydrophillic matrix forming polymer are selected from the group of ethylcellulose, methylcellulose, hydroxypropylcellulose, hydroxymethylcellulose, hydroxypropylmethylcellulose and mixture thereof.

4. A formulation according to claim 1, wherein the best results are obtained by using hydroxymethylcellulose, hydroxypropylcellulose, hydroxypropylmethylcellulose and sodium carboxymethylcellulose with hydroxypropylmethylcellulose (K15M or K100M) being particularly preferred including mixtures thereof.

5. A formulation according to claim 1 wherein the said oral dosage form is a tablet.

6. A formulation acording to claim 1, wherein solid pharmaceutical product is tablet of 200 mg and 400 mg.

7. A formulation according to claim 1, wherein ratio of Nimesulide to hydroxypropylmethylcellulose is between 1:0.1 to 1:0.3

8. A formulation according to claim 1, wherein inert excepient lactose can be replaced by mannitol, sucrose, sorbitol, glucose.

9. A formulation according to claim 1, wherein ratio between Nimesulide and inert excipient varies between 1:0.2 to 1:0.5

10. The formulation as claimed in claim 1 wherein it is in the form selected from suspension, syrups, caplets, powders, lozenges, solutions, granules, capsule, waffers, chewable, occular, intranasal, buccal, sublingual, rectal, and vaginal.

11. A process for the controlled release formulation in the form of oral dosage for treating inflamatory and pain disorders by administering Nimesulide in combination with hyprohilic matrix forming polymer to produce granules together with pharmaceutically acceptable excipients including binders and lubricant tableted as per the commonly used pharmaceutical technology, said oral formulation provides a drug release of nimesulide for extended period of 24 hours.

12. The process for the preperation of controlled release formulation according to claim 1 wherein said hydrophillic matrix forming polymer is selected from alkyl-cellulose hydroxymethylcellulose, hydroxyethylcellulose, hydroxypropylcellulose, hydroxypropylmethylcellulose and sodium carboxymethylcellulose, polyvinylpyrrolidone and mixture thereof.

13. A process for the preperation of controlled release formulation according to claim 1, wherein said hydrophillic matrix forming polymer selected from the group of hydroxypropylcellulose, hydroxymethylcellulose, with hydroxypropylmethylcellulose and mistures thereof. Among this hydroxypropylmethylcellulose bring particularly preferred including mixtures may be used.

14. A process for the preperation of controlled release formulation according to claim 1, wherein the best results are obtained by using hydroxymethylcellulose, hydroxypropylcellulose and hydroxypropylmethylcellulose and sodium carboxymethylcellulose with hydroxypropylmethylcellulose (K15M or K100M) being particularly preferred including mixtures thereof.

15. A process for the preperation of controlled release formulation according to claim 1 wherein the said oral dosage form is a tablet.

16. A process for the preperation of controlled release formulation acording to claim 1, wherein solid pharmaceutical product is tablet of 200 mg and 400 mg.

17. A process for the preperation of controlled release formulation according to claim 1, wherein ratio of Nimesulide to hydroxypropylmethylcellulose is between 1:0.1 to 1:0.3

18. A process for the preperation of controlled release formulation according to claim 1, wherein inert excepient lactose can be replaced by mannitol, sucrose, sorbitol, glucose.

19. A process for the preperation of controlled release formulation according to claim 1, wherein ratio between Nimesulide and inert excipient varies between 1:0.2 to 1:0.5

20. A process for the preperation of controlled release formulation according to claim wherein it is in the form selected from suspension, syrups, caplets, powders, lozenges, solutions, granules, capsule, waffers, chewable, occular, intranasal, buccal, sublingual, rectal, and vaginal.
